# EUROPEAN PATENT APPLICATION

(11) **EP 1 967 151 A1**
(43) Date of publication of application: **10.09.2008**
(21) Application number: 08250630.4
(22) Date of filing: 25.02.2008
(51) Int. Cl.: A61B 17/86, A61B 17/00

(54) **Self tapping screw with resorbable tip**

(30) Priority: 06.03.2007 US 682334
(71) Applicant: Zimmer Technology, Inc., Warsaw, IN 46580 (US)
(72) Inventor: Forstein, Micah, Warsaw, IN 46580 (US); Zhang, Kai, Warsaw, IN 46582 (US)
(74) Representative: Mays, Julie

(57) **Abstract**

A fixation device (2) for use in bone, such as a bone screw, includes a first portion (4) and a second portion (6). The first portion may be manufactured from a short-term resorbable material, and the second portion may be manufactured from a non-resorbable or long term resorbable material. The first portion (4) may comprise a tip capable of self-tapping a bone. The second portion may include at least one thread formed on the outer surface thereof and a head portion formed to mate with a driver.

## Description

### BACKGROUND

### 1. Field of the Invention.

The present invention relates to fixation devices configured for use in the human body. Specifically, the present invention relates to orthopedic screws.

### 2. Description of the Related Art.

Screw type devices configured for use in attaching a medical implant to a bone of a human body are well known. Bone screws are employed for a variety of uses, such as in the attachment of bone plates to a bone. Some bone screws have been configured to self tap threads into the bone when threaded into a pilot hole during insertion of the screw into a bone. Other bone screws have been configured to be resorbed by the body after a suitable period of time.

### SUMMARY

The present invention relates to an orthopedic screw comprising a first portion and a second portion. The first portion comprises a first material and includes a self tapping tip. The second portion may be connected to the first portion and may be comprised of a second material, which may be resorbable or non-resorbable. The first material is configured to be resorbed into a human body at a faster rate than the rate at which the second material may be resorbed into the body.

The first material may comprise at least one of Poly(DL-lactide), Poly(glycolide), Poly(L-lactide-*co*-glycolide) or Poly(DL-lactide-*co*-glycolide). In other embodiments, the first material may comprise at least one of Poly(ethylene glycol)-*co-*polyactide, methyl cellulose, carboxyl methyl cellulose. In embodiments, the first material may comprise at least one of hyaluronic acid, chitosan, collagen gelatin, fibrin, dextran or agarose. In addition, in embodiments, the second material may comprise Poly(L-lactide) or Poly(DL-lactide-*co*-L-lactide).

The inherent viscosity of the first material may have an inherent viscosity as low as about 0.1 or 0.2 dL/g and as high as about 1, 1.5 or 3 dL/g. The second material may have an inherent viscosity as low as about 0.1 or 0.2 dL/g and as high as about 1, 1.5 or 3 dL/g

The second portion may include a head configured to mate with a driver. In addition, the first portion may include at least one thread encompassing an outer surface. The thread encompassing the outer surface may be a starter thread and may include flutes. In embodiments, the second portion may also include a thread. The thread of the second portion may be aligned with the thread of the first portion. The first portion may have a hardness at least equivalent to the hardness of bone.

An advantage of the present invention is the relatively faster resorption of the tip portion of the screw as compared to the remainder of the screw.

A further advantage of the present invention is that the relatively faster resorption of the tip portion of the screw allows the body to heal around the tip portion of the screws while the remainder of the screw secures a bone plate, for example, on a bone.

In one form, the present invention provides an orthopedic screw including a threaded shaft, including a distal portion formed at least in part of a first resorbable material, the distal portion including a self-tapping tip; and a proximal portion formed at least in part of one of a second resorbable material and a non-resorbable material.

In another form, the present invention provides an orthopedic screw including a distal tip portion formed at least in part of a resorbable material; and a threaded shaft portion including a proximal end with a driver interface, the threaded shaft portion formed at least in part of one of a second resorbable material and a non-resorbable material.

### BRIEF DESCRIPTION OF THE DRAWINGS

The above-mentioned and other features and advantages of this invention, and the manner of attaining them, will become more apparent and the invention itself will be better understood by reference to the following description of an embodiment of the invention taken in conjunction with the accompanying drawings, wherein:

Figure 1 is a perspective view of a fixation device;

Figure 2 is a first exploded perspective view of the fixation device of Figure 1;

Figure 3 is a second exploded perspective view of the fixation device of Figure 1;

Figure 4 is a side view of the fixation device shown in Figure 1 in combination with a bone plate configured to be affixed to a bone;

Figure 5 is a side view depicting the insertion of a fixation device through the bone plate and into the bone; and

Figure 6 is a side view depicting a fixation device fully inserted through the bone plate and into the bone with the tip of the fixation device resorbed.

Corresponding reference characters indicate corresponding parts throughout the several views. The exemplification set out herein illustrates one preferred embodiment of the invention, in one form, and such exemplification is not to be construed as limiting the scope of the invention in any manner.

### DETAILED DESCRIPTION

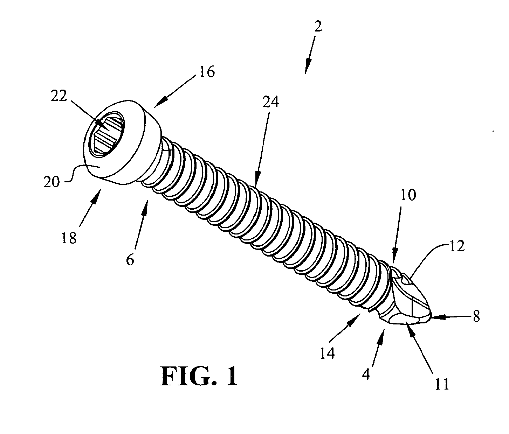

Figure 1 depicts a perspective view of a fixation device 2 representing an embodiment of the present invention. Fixation device 2 may be a bone screw or a nail, for example, and includes a first, or distal, portion 4 and a second, or proximal, portion 6. In the depicted embodiment, first portion 4 represents the tip of the bone screw 2, and second portion 6 represents the body the bone screw 2.

In the present embodiment, first portion 4 includes a first end 8 and a second end 10. First portion 4 further includes a plurality of flutes, each generally indicated by numeral 11, and a thread, generally indicated by numeral 12. Flutes 11 may have any configuration known in the art and are generally shaped for self tapping into a bone. Thread 12, which may be referred to as a starter thread, encompasses a portion of the outer surface of first portion 4 and is capable of forming threads in a bone. Accordingly, first portion 4, including thread 12, is generally manufactured from a material having a hardness greater than that of bone. In embodiments, the flutes 11 may interrupt the travel of the thread 12 around the first portion 4.

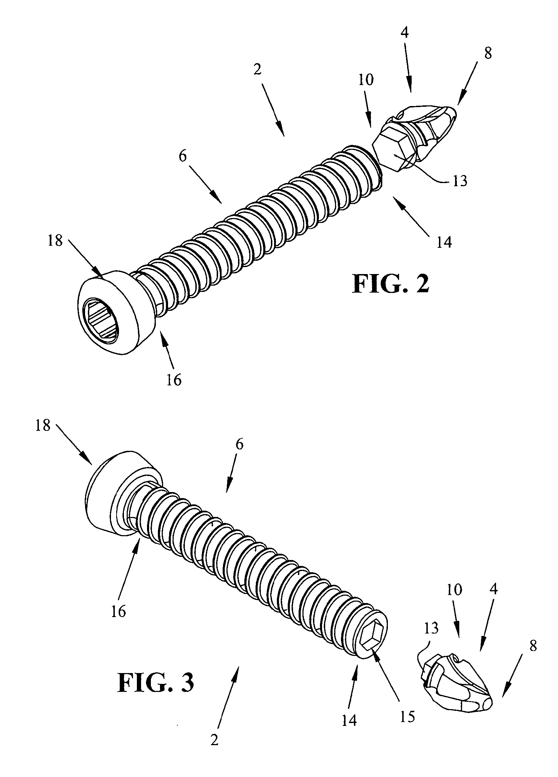

Second portion 6 includes a first end 14 and a second end 16. In the present embodiment, first end 14 is configured to mate with second end 10 of first portion 4 in a suitable manner, such that first and second portions 4 and 6 may be rotatably driven as a unit. For example, in embodiments of the invention, as shown in Figures 2 and 3, second end 10 includes a hexagonal protrusion 13, and first end 14 includes a complementary hexagonal recess 15 or vice-versa. Recess 15 may receive protrusion 13 when mating first portion 4 to second portion 6. Other suitable configurations may be employed as necessary to mate the two portions 4, 6.

In addition, in embodiments, first end 14 may be affixed to second end 10 by way of a biocompatible adhesive, for example. In embodiments, the ends 10, 14 may be conjoined via a welding process, for example.

Referring again to Figure 1, second end 16 includes a driver mating portion 18. In the present embodiment, driver mating portion 18 comprises a head 20 including a recess 22. Head 20 may have any profile suitable for the intended usage of the device 2. For example, head 20 may be semi-spherical or spherical.

In the depicted embodiment, second portion 6 includes a driver interface, such as recess 22, which is configured to receive a driver (not shown) known in the art. Recess 22 may have any known shape complementary to that of drivers typically employed in the art. In other embodiments, driver mating portion 18 may take any known configuration capable of mating with a driver. For example, mating portion 18 may include protrusions, not shown, configured to be received by a driver tool. In other embodiments, mating portion 18 may include at least one recess configured to receive protrusions of the driver tool.

Second portion 6 further includes a thread, generally indicated by numeral 24. In the depicted embodiment, thread 24 extends from first end 14 toward second end 16 to a position proximate driver mating portion 18. In addition, in the depicted embodiment, thread 12 may gradually transition to thread 24 and the threads 12, 24 may be complementary. For example, the threads 12, 24 may have a uniform pitch and may be like handed, i.e. the threads 12, 24 may share the same direction of advancement upon rotation.

First portion 4 may be resorbed within hours or days by way of any known process, such as through a bio-resorption process or through a galvanic corrosion process. First portion 4 may also be manufactured from any known material generally having a hardness greater than bone and capable of being resorbed by a human body. For example, first portion 4 may be manufactured from synthetic polymers such as Poly(DL-lactide), Poly(glycolide), Poly(L-lactide-*co*-glycolide) or Poly(DL-lactide-*co*-glycolide). Additional materials may also be utilized in forming first portion 4, such as polymer blends comprising any combination of the above polymers. Synthetic hydrogel materials, such as Poly(ethylene glycol)-*co*-polylactide, methyl cellulose, or carboxy methyl cellulose may also be used in forming first portion 4. In addition, natural biopolymers including anionic biopolymers, such as hyaluronic acid, cationic bioploymers, such as chitosan, amphipathic polymers, such as collagen, gelatin and fibrin, and neutral polysaccharides, such as dextran and agarose.

The inherent viscosity of the first portion 4 following final processing and sterilization may be as low as about 0.1 or 0.2 dL/g and as high as about 1, 1.5 or 3 dL/g.

Second portion 6 may be manufactured from any material suitable for the desired application. The material comprising second portion 6 may be manufactured from a non-resorbable material, such as a biocompatible plastic, titanium, including Ti 6-4 ELI, or stainless steel, including grade 316L, or from a long term resorbable material that may not be resorbed by a human body for many days or months. For example, second portion 6 may be manufactured from synthetic polymers such as Poly(L-lactide) and Poly(DL-lactide-*co*-L-lactide). In addition, second portion 6 may be manufactured from blends of these synthetic polymers. Furthermore, composites materials including calcium phosphate fillers, such as hydroxylapatite or tricalcium phosphate, in the form of particulate or fibers may be utilized with the any of the above polymers or polymer blends.

The inherent viscosity of the second portion 6 following final processing and sterilization may be as low as about 0.1 or 2 dL/g and as high as about 5 or 10 dL/g.

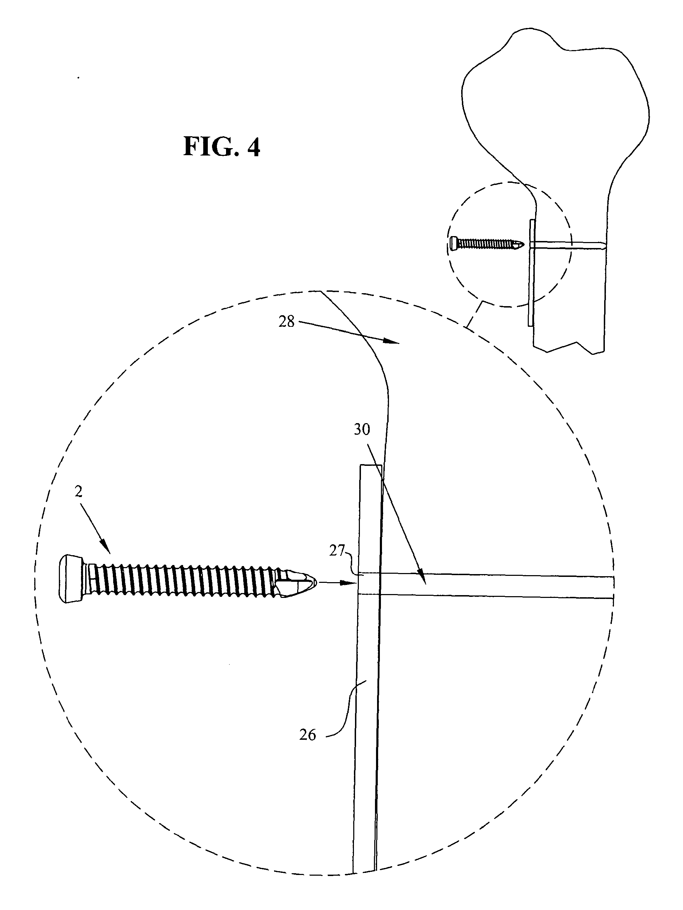

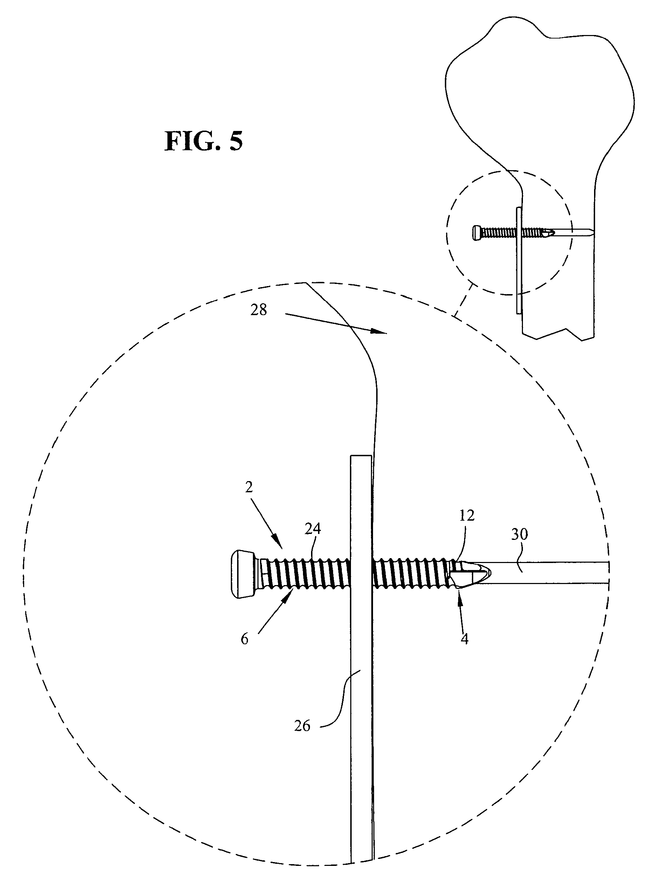

Figures 4 through 6 depict an exemplary use of a fixation device 2, namely, for attaching a bone plate 26 to a bone 28. As shown in the Figures, bone plate 26 has a shape complementary to the contour of the surface of bone 28. Plate 26 also includes a through hole 27 sized to receive fixing device 2. As shown in Figure 5, plate 26 may be affixed to bone 28 with a known temporary fastener (not shown). A pilot hole 30 may be formed in bone 28 in a known manner to align with the position of the through hole of plate 26. The side wall of pilot hole 30 is depicted as substantially smooth but may also be threaded.

Fixing device 2 may then be inserted into the through hole of plate 26 and into pilot hole 30, as depicted in Figure 4. Pilot hole 30 is sized so that fixing device 2 contacts the side wall of the pilot hole 30.

As shown in Figure 5, upon rotation of fixing device 2, the flutes 11 cut into the bone material thereby increasing the size of pilot hole 30. In addition, thread 12 cuts into the side wall of pilot hole 30 forming a trace that corresponds to the threads 12, 24 of device 2. As the device 2 continues to traverse pilot hole 30, threads 12 continue to cut into the side wall of pilot hole 30, and threads 24 continue to traverse the newly formed cuts.

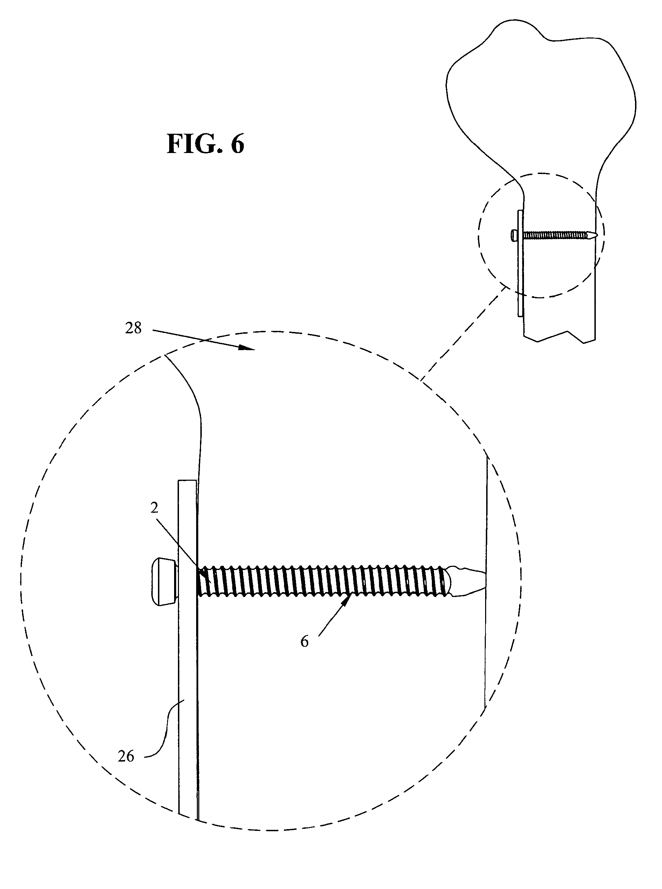

Once device 2 has been fully inserted, first portion 2 may be quickly resorbed by the body leaving only second portion 6 to maintain plate 26 in its position relative to bone 28, as shown in Figure 6. Advantageously, the relatively quick resorption of the first portion 4 will allow tissue proximate first portion 4 to more rapidly heal while second portion 6 still connects the plate 26 to the bone 28.

While this invention has been described as having exemplary designs, the present invention may be further modified within the spirit and scope of the disclosure. This application is therefore intended to cover any variations, uses, or adaptations of the invention using its general principles. Further, this application is intended to cover such departures from the present disclosure as come within known or customary practice in the art to which this invention pertains.

## Claims

**1.** An orthopedic screw, comprising:
a threaded shaft, comprising:
a distal portion formed at least in part of a first resorbable material, said distal portion including a self-tapping tip; and
a proximal portion formed at least in part of one of a second resorbable material and a non-resorbable material.

**2.** The orthopedic screw of claim 1, wherein said second portion is formed of a second resorbable material, and said first resorbable material is resorbable into a human body at a faster rate than said second resorbable material.

**3.** The orthopedic screw of claim 1 wherein said second material is selected from the group consisting of Poly(L-lactide) and Poly(DL-lactide-*co*-L-lactide).

**4.** The orthopedic screw of claim 1 wherein said second material is selected from the group consisting of titanium or stainless steel.

**5.** The orthopedic screw of claim 1 wherein said first material is selected from the group consisting of Poly(DL-lactide), Poly(glycolide), Poly(L-lactide-*co*-glycolide) and Poly(DL-lactide-*co*-glycolide).

**6.** The orthopedic screw of claim 1 wherein said first material is selected from the group consisting of Poly(ethylene glycol)-*co*-polylactide, methyl cellulose, and carboxy methyl cellulose.

**7.** The orthopedic screw of claim 1 wherein said first material is selected from the group consisting of hyaluronic acid, chitosan, collagen, gelatin, fibrin, dextran and agarose.

**8.** The orthopedic screw of claim 1 wherein said second portion includes a head configured to mate with a driver.

**9.** The orthopedic screw of claim 1 wherein said first portion includes at least a portion of a thread.

**10.** The orthopedic screw of claim 1 wherein said first material has an inherent viscosity within the range of about 0.1-3 dL/g.

**11.** The orthopedic screw of claim 1 wherein said second material has an inherent viscosity within the range of about 0.1-10 dL/g.

**12.** The orthopedic screw of claim 1 wherein said first portion is non-rotatably mated to said second portion.

**13.** The orthopedic screw of claim 1 wherein said first portion is joined to said second portion by an adhesive.

**14.** The orthopedic screw of claim 1 wherein said first portion is joined to said second portion by way of welding.

**15.** An orthopedic screw, comprising:
a distal tip portion formed at least in part of a resorbable material; and
a threaded shaft portion including a proximal end with a driver interface, said threaded shaft portion formed at least in part of one of a second resorbable material and a non-resorbable material.

**16.** The orthopedic screw of claim 15, wherein said second portion is formed of a second resorbable material, and said first resorbable material is resorbable into a human body at a faster rate than said second resorbable material.

**17.** The orthopedic screw of claim 15 wherein said first portion is non-rotatably mated to said second portion.

**18.** The orthopedic screw of claim 15 wherein said second material is selected from a group consisting of Poly(L-lactide) and Poly(DL-lactide-*co*-lactide).

**19.** The orthopedic screw of claim 15 wherein said second material is selected from the group consisting of titanium or stainless steel.

**20.** The orthopedic screw of claim 15 wherein said first material has an inherent viscosity within the range of about 0.1-3 dL/g.

**21.** The orthopedic screw of claim 15 wherein said second material has an inherent viscosity within the range of about 0.1-10 dL/g.

**22.** The orthopedic screw of claim 15 wherein said first material is selected from the group consisting of Poly(DL-lactide), Poly(glycolide), Poly(L-lactide-*co*-glycolide) and Poly(DL-lactide-*co*-glycolide).

**22.** The orthopedic screw of claim 15 wherein said first material is selected from the group consisting of Poly(ethylene glycol)-*co*-polylactide, methyl cellulose, and carboxy methyl cellulose.

**24.** The orthopedic screw of claim 15 wherein said first material is selected from the group consisting of hyaluronic acid, chitosan, collagen, gelatin, fibrin, dextran and agarose.
